# EUROPÄISCHE PATENTSCHRIFT

(11) **EP 2 917 177 B1**
(45) Veröffentlichungstag und Bekanntmachung des Hinweises auf die Patenterteilung: **06.12.2017**
(21) Anmeldenummer: 14758783.6
(22) Anmeldetag: 22.08.2014
(51) Int. Cl.: C07C 321/04, C07C 403/24, A61K 31/198

(54) **BETA-CAROTIN ZUBEREITUNG**
BETA CAROTENE PREPARATION
PRÉPARATION DE BÊTA-CAROTÈNE

(30) Priorität: 02.09.2013 AT 6802013
(43) Veröffentlichungstag der Anmeldung: 16.09.2015
(60) Teilanmeldung: 17197925.5
(73) Patentinhaber: Inpharsearch AG, 6300 Zug (CH)
(72) Erfinder: FRANTSITS, Werner J., A-1130 Wien (AT)
(74) Vertreter: Beer & Partner Patentanwälte KG
(86) Internationale Anmeldenummer: PCT/EP2014/002310
(87) Internationale Veröffentlichungsnummer: WO 2015/028132

(56) Entgegenhaltungen:
- EP-A1- 1 016 404
- DE-A1- 19 609 477
- DE-A1- 19 819 616

## Beschreibung

Die Erfindung betrifft eine Zubereitung von Beta-Carotin, insbesondere eine wässerige Emulsion von Beta-Carotin, die zur parenteralen Verabreichung geeignet ist (DE 196 09 477 A1).

Aus der EP 1 016 404 A1 (AT 408 186 B) ist eine wässerige Zubereitung von Beta-Carotin bekannt, die in der Veterinärmedizin verwendbar ist. Diese wässerige Zubereitung von Beta-Carotin, in der Beta-Carotin als micellare Lösung (Mikroemulsion) vorliegt, enthält anionische oder nichtionische Lösungsvermittler, beispielsweise Polyoxythylen-660-Hydroxystearat und/oder Isopropylmyristat. Die Zubereitung, die beispielsweise 0,1 bis 10 Prozent (w/v) Beta-Carotin enthält, kann weiters Antioxidantien und wenigstens ein Konservierungsmittel enthalten.

Eine in der Veterinärmedizin verwendbare Injektionslösung aus Mehrfachdosisbehältern, die Beta-Carotin enthält, wird von Alvetra und Werfft in Wien unter dem Markennamen "Carofertin" auf den Markt gebracht. Die bekannte Injektionslösung enthält je Milliliter Injektionslösung 10,0 mg Beta-Carotin, 10,0 mg Benzylalkohol, 0,12 mg Ascorbylpalmitat, 0,10 mg Alpha-Tocopherol, Macrogol-15-hydroxystearat (Solutol HS 15), Isopropylmyristat und Wasser für Injektionszwecke.

Bekannt ist, dass Beta-Carotin im Organismus auf zweierlei Weisen wirkt. Einerseits wird es als Provitamin in Vitamin A umgewandelt und entfaltet über diesen metabolischen Schritt seine Wirkung, andererseits greift Beta-Carotin selbst aktiv in den Stoffwechsel ein. Beta-Carotin hat einen stabilisierenden Effekt auf die Corpora lutea der Ovarien, bewirkt die Stimulation des Follikelwachstums und hat einen protektiven und entzündungshemmenden Einfluss auf das Endometrium.

Die parenterale Verabreichung von Beta-Carotin beim Schwein eine Woche vor der Deckung erhöht die Wurfgröße. Dieser Effekt ist besonders deutlich bei Alt-Säuen ausgeprägt.

Eine ausreichende Versorgung von Muttertieren mit Beta-Carotin bewirkt außerdem eine deutliche Immunitätssteigerung der Neugeborenen, mit verminderter Anfälligkeit gegen Darminfektionen und somit geringe Verluste in der Aufzuchtphase.

Problematisch bei bekannten Emulsionen von Beta-Carotin ist es, dass das Konservierungssystem nur relativ kurze Zeit, nämlich 12 bis maximal 15 Monate, in geschlossenem Zustand des Behälters stabil ist und nach erstmaligen Anbruch der Injektionslösung bereits innerhalb weniger Tage zusammenbricht, womit der Abbau des Konservierungsmittels und damit der des Wirkstoffes die zugelassenen Grenzen überschreitet.

Insoweit war die Verwendbarkeit der bekannten Beta-Carotin-Emulsionen in der Tiermedizin begrenzt, als eine zu kurze Haltbarkeit zu beachten war, was in der tierärztlichen Praxis zu häufigem Verwerfen von nur wenig angebrauchten Injektionsflaschen und damit zu unnötigen Verlusten führt.

In der EP 1 016 404 A1 und der AT 408 186 B ist erwähnt, dass Emulsionen von Beta-Carotin problematisch sind, da sie eine nur geringe Stabilität gegenüber spontan einsetzender Phasentrennung besitzen. Weiters ist die Beständigkeit von Beta-Carotin gegen Oxidation durch in der Luft enthaltenen Sauerstoff in Emulsionen schlecht.

Die DE 196 09 477 A1 beschreibt wässerige Solubilisate, die zur parenteralen Verabreichung geeignet sind, welche zumindest ein Carotinoid, zumindest ein nicht wasserlösliches Vitamin und einen nichtionogenen Emulgator enthalten. In einer bevorzugten Ausführungsform enthält die Formulierung neben dem Carotinoid (einen) Tocopherol(ester), Ascorbinsäure sowie gegebenenfalls N-Acetylcystein. Als nichtionogener Emulgator wird Polyoxyethylen-12-hydroxystearat genannt. Die aus DE 196 09 477 A1 bekannte Formulierung ist zum sofortigen Verbrauch bestimmt und für das Bereitstellen in einem Behältnis für Mehrfachdosis nicht geeignet.

Die DE 198 19 616 A1 offenbart eine Zusammensetzung zur Behandlung/Prophylaxe entzündlicher Hautkrankheiten, die N-Acetylcystein und zumindest ein weiteres Antioxidationsmittel ausgewählt aus der Gruppe Ascorbinsäure, α-Tocopherol, β-Carotin und/oder Derivate selbiger enthält. Die Zusammensetzung kann parenteral verabreicht werden und ist nur für das Bereitstellen in einem Behältnis für Einmaldosis geeignet.

Gegenstand der DE 197 47 546 A1 ist die Verwendung von systemisch verabreichten, wasserlöslichen Antioxidantien (z. B. Ascorbat, N-Acetylcystein) gemeinsam mit lipidlöslichen Antioxidantien (z. B. Carotinoide, Tocopherol) zur Behandlung entzündlicher Dermatosen. Diese Zubereitung ist nur für das Bereitstellen in einem Behältnis für als Einmaldosis geeignet.

Der Erfindung liegt die Aufgabe zugrunde, eine insbesondere zur parenteralen Verabreichung, vor allem in der Veterinärmedizin, geeignete wässerige Zubereitung von Beta-Carotin einerseits und ein Verfahren zum Herstellen derselben andererseits zur Verfügung zu stellen, wobei die Zubereitung in Mehrdosisbehältnissen bereitgestellt werden kann.

Gelöst wird diese Aufgabe erfindungsgemäß mit einer Zubereitung mit den Merkmalen von Anspruch 1.

Insoweit das Herstellungsverfahren betroffen ist, wird die der Erfindung zugrunde liegende Aufgabe mit den im unabhängigen, auf das Herstellungsverfahren gerichteten, Anspruch genannten Merkmalen gelöst.

Der Erfindung liegt die überraschende Erkenntnis zugrunde, dass der Zusatz von Acetylcystein zu Emulsionen, die Beta-Carotin enthalten, einen stark stabilisierenden Einfluss auf das Konservierungssystem ausübt und damit die Stabilität der Emulsion verbessert, wenn die Zubereitung ein Antioxidans und als Konservierungsmittel Benzylalkohol enthält.

Acetylcystein (L-∝-Acetamido-ß-mercaptoproprionsäure) ist ein an sich bekannter Stoff, insbesondere ein Arzneistoff, der als Expektorans bei Atemwegserkrankungen und als Antidot bei Paracetamol-Intoxikation eingesetzt wird.
Acetylcystein wird auch in der Nephrologie, bei Infektionskrankheiten und in der Psychiatrie angewendet. Acetylcystein ist weder als Stabilisator noch als Konservierungsmittel oder Lösungsvermittler bekannt, so dass der oben geschilderte Effekt der Stabilisierung von Beta-Carotin enthaltenden Emulsionen überraschend ist.
Erfindungsgemäße Emulsionen von Beta-Carotin, die zur parenteralen Verabreichung geeignet sind, sind in ungeöffneten Behältnissen (z. B. Mehrdosisbehältnissen) Jahre und nach erstem Anbruch mindestens 8 Wochen stabil, was es erlaubt, die Emulsion zur Fertilitätssteigerung, insbesondere bei Rind und Schwein, sowie zum Behandeln von Störungen im Fertilitätstrakt bei Hunden einzusetzen.
Im Rahmen der Erfindung ist insbesondere in Betracht gezogen, dass der Gehalt von Acetylcystein im Bereich von 1 Gew.% bis 8 Gew.%, beispielsweise bei 3 Gew.%, bezogen auf die Emulsion liegt.
Weitere mögliche Bestandteile der erfindungsgemäßen, wässerigen Emulsion von Beta-Carotin sind neben Beta-Carotin und Benzylalkohol insbesondere Ascorbylpalmitat, Alpha-Tocopherol, Lösungsvermittler wie Isopropylmyristat, wenigstens ein nicht-ionischer Lösungsvermittler, wie Macrogol-15-hydroxystearat (Solutol HS15), und Wasser.

Eine erfindungsgemäße wässerige Emulsion von Beta-Carotin enthält bevorzugt Beta-Carotin je 1000 g Emulsion in Mengen von 5 bis 15 g, insbesondere in einer Menge von 10 g je 1000g Emulsion.
Die als Antioxidans gegebenenfalls eingesetzten Wirkstoffe Ascorbylpalmitat und Alpha-Tocopherol werden in Mengen von 0,05 bis 0,50 g, vorzugsweise 0,12g (Ascorbylpalmitat) und 0,05 bis 0,20 g, vorzugsweise 0,10g (Alpha-Tocopherol) je 1000 g Emulsion eingesetzt.
Das als weiterer Lösungsvermittler gegebenenfalls zugesetzte Isopropylmiristat kann in Mengen von 70 bis 90 g, insbesondere 84 g je 1000 g Emulsion enthalten sein.
Das die Emulsion stabilisierende und Beta-Carotin vor Abbau schützende Acetylcystein (Konservierungsmittel) kann in Mengen zwischen 1 und 8 g, insbesondere 3 g, je 1000 g Emulsion vorliegen.
Beim erfindungsmäßigen Verfahren zum Herstellen der erfindungsgemäßen wässerigen Emulsion von Beta-Carotin, wird der Schritt des Zusetzens von Isopropylmyristat zu dem, beispielsweise geschmolzenen und vorzugsweise nicht-ionischen, Lösungsvermittler (z. B. Solutol) bei einer Temperatur zwischen 25° Celsisus und 40° Celsius, vorzugsweise bei 30° Celsius, ausgeführt.
Nachstehend werden weiteren Einzelheiten der Erfindung, an Hand eines Ausführungsbeispieles beschrieben.

### Beispiel 1:

Eine wässerige Emulsion von Beta-Carotin, die in der Veterinärmedizin zur parenteralen Verabreichung geeignet ist, hat die folgende Zusammensetzung:
Beta-Carotin 10,0 g, Ascorbylpalmitat 0,12 g, Alpha-Tocopherol 0,10 g, Benzylalkohol 10,0 g, Isopropylmyristat 84,0 g, Acetylcystein 3,0 g, Solutol HS 15 182,0 g, Wasser 711,0 g. Zusammen 1000,0 g.
   Zusammen 1000,0 g.

### Beispiel 5:

Die in Beispiel 1 beschriebene wässrige Emulsion von Beta-Carotin kann wie folgt hergestellt werden:
Als nicht-ionischer Lösungsvermittler wird Solutol HS 15 (2-Hydroxyethyl-12-hydroxyoktadekanoat, Macrogol-15-hydroxystearat) im Ansatzkessel auf 60° Celsius erwärmt, schmilzt und ist dann dünnflüssig.

Zu dem geschmolzenen Solutol HS 15 wird flüssiges Isopropylmyristat zugegeben. Das Gemisch wird unter mäßigen Rühren auf 130° Celsius erhitzt.

Der so erhaltenen Lösung wird bei erhöhter Temperatur der Lösung und unter mäßigem Rühren Beta-Carotin langsam zugesetzt und so lange weitergerührt, bis eine dunkelrote, klare Lösung vorliegt. Dabei kann unter Stickstoffatmosphäre gearbeitet werden.

Während dieser Arbeitsschritte (Zugeben der öligen Mischung von Beta-Carotin und nachfolgendem Rühren) wird eine Temperatur von 90° Celsius eingehalten. Dabei kann unter Stickstoffatmosphäre gearbeitet werden.

In einem gesonderten Behältnis wird bei Raumtemperatur Ascorbylpalmitat und Alpha-Tocopherol in Benzylalkohol gelöst.

Nachdem die so erhaltene Beta-Carotin enthaltende Emulsion langsam auf 50° Celsius abgekühlt worden ist, wird die zuvor erhaltene Lösung von Ascorbylpalmitat, Alpha-Tocopherol in Benzylalkohol unter Rühren in die Emulsion eingebracht. Auch bei diesem Schritt wird bevorzugt unter Stickstoffatmosphäre gearbeitet.

Als nächster Schritt wird Acetylcystein bei einer Temperatur von 30° Celsius in Wasser gelöst.

Sobald die zuvor erhaltene Beta-Carotin enthaltende Emulsion auf 30° Celsius abgekühlt ist, wird die Acetylcysteinlösung, bevorzugt unter Stickstoffatmosphäre, langsam in die Emulsion eingerührt und solange mäßig gerührt, bis eine dunkelrote klare Emulsion vorliegt.

In den nachstehenden Tabellen sind die Ergebnisse von Stabilitätsprüfungen wiedergegeben.

**Tabelle I:**

| Beta-Carotin-Zubereitung gemäß AT 408 186 B1: | | | |
|---|---|---|---|
| | Gehalte nach Produktion | Nach 6 Monaten | Nach 12 Monaten |
| Beta-Carotin (all-trans + cis) | 99,5 - 101 % | 97,5 - 99 % | 96 - 97 % |
| Benzylalkohol | 100 - 101,5 % | 78 - 86 % | 62 - 65 % |
| Ascorbinpalmitat | 99 - 101 % | 69 - 74 % | 58 - 64 % |
| Tocopherol | 99 - 101 % | 98 - 99 % | 95 - 97 % |

**Tabelle II:**

| Erfindungsgemäße Beta-Carotin-Zubereitung: Beispiel 1: | | | |
|---|---|---|---|
| | Gehalte nach Produktion | Nach 6 Monaten | Nach 12 Monaten |
| Beta-Carotin (all-trans+cis) | 99,5 - 101 % | 99,5 - 100,5 % | 99 - 100 % |
| Benzylalkohol | 100 - 101,5 % | 99,5 - 100 % | 98 - 99,5 % |
| Ascorbinpalmitat | 99 - 101 % | 98 - 100 % | 94 - 97 % |
| Tocopherol | 99 - 101 % | 99 - 100,5 % | 97 - 99,5 % |

Der in Tabelle II nicht genannte Gehalt an Acetylcystein entspricht dem in Beispiel 1 genannten Gehalt. Bei den Stabilitätsprüfungen waren die Sample-Größen je Versuchsansatz 85 Liter, abgefüllt in 100 ml Durchstichflaschen (ungeöffnet), eingesetzt.

Da in der Tiermedizin Zimmertemperatur als Lagerungsbedingung gewünscht ist, wurden für die Stabilitätsuntersuchung folgende Lagerungsbedingungen eingehalten:
25°Celsius bei 60 % Luftfeuchtigkeit.

Aus den oben, in Tabelle I wiedergegebenen Stabilitätsdaten einer bekannten Beta-Carotin-Zubereitung und in Tabelle II wiedergegebenen Stabilitätsdaten einer erfindungsgemäßen Beta-Carotin-Zubereitung ist ersichtlich, dass der Zusatz von Acetylcystein nicht nur Beta-Carotin, sondern auch den in der Zubereitung enthaltenen Anteil an Konservierungsmitteln (das "Konservierungssystem") vor Abbau schützt, also die Stabilität der Zubereitung erhöht.

## Patentansprüche

1. Zubereitung von Beta-Carotin in einem wässerigen Medium, wobei die Zubereitung als Emulsion zur parenteralen Verabreichung vorliegt und Acetylcystein enthält, **dadurch gekennzeichnet, dass** die Zubereitung wenigstens ein Antioxidans, insbesondere Ascorbylpalmitat und/oder Alpha-Tocopherol, sowie als Konservierungsmittel Benzylalkohol, enthält.

2. Zubereitung nach Anspruch 1, **dadurch gekennzeichnet, dass** Acetylcystein bezogen auf die Emulsion in Mengen zwischen 1 und 8 Gew.% vorzugsweise 2 bis 5 Gew.%, besonders bevorzugt 3 Gew.%, vorliegt.

3. Zubereitung nach Anspruch 1 oder 2, **dadurch gekennzeichnet, dass** die Emulsion einen Lösungsvermittler, insbesondere Isopropylmyristat, enthält.

4. Zubereitung nach einem der Ansprüche 1 bis 3, **dadurch gekennzeichnet, dass** die Emulsion wenigstens einen nicht-ionischen Lösungsvermittler, insbesondere Hydroxyethyl-12-hydroxyoktadekanoat und Macrogol-15-hydroxystearat, (Solutol HS 15) enthält.

5. Verfahren zum Herstellen einer Emulsion nach Anspruch 3 oder 4, **gekennzeichnet durch** folgende Verfahrensschritte:
a) Erwärmen des nicht-ionischen Lösungsvermittlers, insbesondere des Hydroxyethyl-12-hydroxyoktadekanoat , um den nicht-ionischen Lösungsvermittler zu schmelzen, und Zusetzen von Isopropylmyristat zu dem geschmolzenen Lösungsvermittler,
b) Einrühren von Beta-Carotin in die in Schritt a) erhaltene Lösung,
c) Eintragen der in Schritt b) erhaltenen Beta-Carotin enthaltende Mischung in Wasser unter Rühren,
d) Rühren des in Schritt c) erhaltenen Gemisches, bis eine dunkelrote, klare Emulsion vorliegt,
e) Lösen von wenigstens einem Antioxidans in Benzylalkohol, der gleichzeitig als Konservierungsmittel dient,
f) Einrühren der in Schritt e) erhaltenen Lösung des Antioxidans in die, in Schritt d) erhaltene Beta-Carotin enthaltende Emulsion und
g) Zugeben von Acetylcystein in die in Schritt f) erhaltene Emulsion.

6. Verfahren nach Anspruch 5, **dadurch gekennzeichnet, dass** der nicht-ionische Lösungsvermittler auf eine Temperatur zwischen 135° Celsius und 140° Celsius erhitzt wird, bevor Isopropylmyristat zugesetzt wird.

7. Verfahren nach Anspruch 6, **dadurch gekennzeichnet, dass** dem geschmolzenen Lösungsvermittler Isopropylmyristat bei einer Temperatur zwischen 125 und 140° Celsius, insbesondere bei 130° Celsius, zugesetzt wird.

8. Verfahren nach Anspruch 6 oder 7, **dadurch gekennzeichnet, dass** die im Schritt b) erhaltene Mischung enthaltend Beta-Carotin, den geschmolzenen nicht-ionischen Lösungsvermittler und Isopropylmyristat in erwärmtes Wasser eingetragen und gerührt wird, bis eine dunkelrote klare Emulsion vorliegt.

9. Verfahren nach einem der Ansprüche 5 bis 8, **dadurch gekennzeichnet, dass** in Schritt b) bei einer Temperatur von 90° Celsius gerührt wird.

10. Verfahren nach einem der Ansprüche 5 bis 9, **dadurch gekennzeichnet, dass** der in Schritt d) erhaltenen Beta-Carotin enthaltenden Emulsion eine Lösung wenigstens eines Antioxidans bei einer Temperatur von etwa 50° zugesetzt wird.

11. Verfahren nach Anspruch 10, **dadurch gekennzeichnet, dass** der mit Antioxidans versetzten Emulsion bei einer Temperatur von 30° Celsius Acetylcystein, insbesondere in Wasser gelöstes Acetylcystein, zugemischt wird.

12. Verfahren nach einem der Ansprüche 5 bis 11 **dadurch gekennzeichnet, dass** die Verfahrensschritte, mit Ausnahme des ersten Verfahrensschrittes a), unter Stickstoffatmosphäre ausgeführt werden.

## Claims

1. Preparation of beta carotene in an aqueous medium, whereby the preparation is present as an emulsion for parenteral administration and contains acetylcysteine, **characterized in that** the preparation contains at least one antioxidant, in particular ascorbyl palmitate and/or alpha-tocopherol, as well as benzyl alcohol as a preservative.

2. Preparation according to Claim 1, **characterized in that** acetylcysteine is present relative to the emulsion in amounts of between 1 and 8% by weight, preferably 2 to 5% by weight, and especially preferably 3% by weight.

3. Preparation according to Claim 1 or 2, **characterized in that** the emulsion contains a solubilizer, in particular isopropyl myristate.

4. Preparation according to one of Claims 1 to 3, **characterized in that** the emulsion contains at least one non-ionic solubilizer, in particular hydroxyethyl-12-hydroxyoctadecanoate and macrogol-15-hydroxystearate (Solutol HS 15).

5. Method for the production of an emulsion according to one of Claims 3 or 4, **characterized by** the following process steps:
a) Heating the non-ionic solubilizer, in particular the hydroxyethyl-12-hydroxyoctadecanoate, in order to melt the non-ionic solubilizer, and adding isopropyl myristate to the molten solubilizer,
b) Stirring beta carotene into the solution obtained in step a),
c) Introducing the beta-carotene-containing mixture that is obtained in step b) into water while being stirred,
d) Stirring the mixture that is obtained in step c) until a dark red, clear emulsion is present,
e) Dissolving at least one antioxidant in benzyl alcohol, which simultaneously serves as a preservative,
f) Stirring the solution of the antioxidant, obtained in step e), into the beta-carotene-containing emulsion that is obtained in step d), and
g) Adding acetylcysteine into the emulsion that is obtained in step f).

6. Method according to Claim 5, **characterized in that** the non-ionic solubilizer is heated to a temperature of between 135° Celsius and 140° Celsius before isopropyl myristate is added.

7. Method according to Claim 6, **characterized in that** isopropyl myristate is added to the molten solubilizer at a temperature of between 125 and 140° Celsius, in particular at 130° Celsius.

8. Method according to Claim 6 or 7, **characterized in that** the mixture that contains beta carotene, molten non-ionic solubilizer and isopropyl myristate that is obtained in step b) is introduced into heated water and stirred until a dark red, clear emulsion is present.

9. Method according to one of Claims 5 to 8, **characterized in that** the stirring in step b) is at a temperature of 90° Celsius.

10. Method according to one of Claims 5 to 9, **characterized in that** the beta-carotene-containing emulsion that is obtained in step d) is added to a solution of at least one antioxidant at a temperature of approximately 50°.

11. Method according to Claim 10, **characterized in that** the emulsion that is mixed with antioxidant is mixed in at a temperature of 30° Celsius with acetylcysteine, in particular acetylcysteine that is dissolved in water.

12. Method according to one of Claims 5 to 11, **characterized in that** the process steps, with the exception of the first process step a), are carried out under nitrogen atmosphere.

## Revendications

1. Préparation de bêta-carotène dans un milieu aqueux, la préparation se présentant sous la forme d'une émulsion destinée à l'administration par voie parentérale et contenant de l'acétylcystéine, **caractérisée en ce que** la préparation contient au moins un antioxydant, en particulier du palmitate d'ascorbyle et/ou de l'alpha-tocophérol, ainsi que de l'alcool benzylique comme conservateur.

2. Préparation selon la revendication 1, **caractérisée en ce que** l'acétylcystéine est présente dans une quantité comprise entre 1 et 8 % en poids, de préférence de 2 à 5 % en poids, plus préférentiellement de 3 % en poids par rapport à l'émulsion.

3. Préparation selon la revendication 1 ou 2, **caractérisée en ce que** l'émulsion contient un agent solubilisant, en particulier du myristate d'isopropyle.

4. Préparation selon l'une des revendications 1 à 3, **caractérisée en ce que** l'émulsion contient au moins un agent solubilisant non ionique, en particulier du 12-hydroxyoctadécanoate d'hydroxyéthyle et de l'hydroxystéarate de macrogol 15 (Solutol HS 15).

5. Procédé de fabrication d'une émulsion selon la revendication 3 ou 4, **caractérisé par** les étapes suivantes :
a) chauffage de l'agent solubilisant non ionique, en particulier du 12-hydroxyoctadécanoate d'hydroxyéthyle, pour faire fondre l'agent solubilisant non ionique, et ajout de myristate d'isopropyle à l'agent solubilisant fondu,
b) délayage de bêta-carotène dans la solution obtenue à l'étape a),
c) introduction du mélange contenant du bêta-carotène obtenu à l'étape b) dans de l'eau sous agitation,
d) agitation du mélange obtenu à l'étape c) jusqu'à obtention d'une émulsion claire rouge foncé,
e) dissolution d'au moins un antioxydant dans de l'alcool benzylique qui sert en même temps de conservateur,
f) délayage de la solution d'antioxydant obtenue à l'étape e) dans l'émulsion contenant du bêta-carotène obtenue à l'étape d) et
g) ajout d'acétylcystéine dans l'émulsion obtenue à l'étape f).

6. Procédé selon la revendication 5, **caractérisé en ce que** l'agent solubilisant non ionique est chauffé à une température comprise entre 135° Celsius et 140° Celsius avant l'ajout de myristate d'isopropyle.

7. Procédé selon la revendication 6, **caractérisé en ce que** le myristate d'isopropyle est ajouté à l'agent solubilisant fondu à une température comprise entre 125 et 140° Celsius, en particulier à 130° Celsius.

8. Procédé selon la revendication 6 ou 7, **caractérisé en ce que** le mélange contenant du bêta-carotène, l'agent solubilisant fondu non ionique et du myristate d'isopropyle obtenu à l'étape b) est introduit dans de l'eau chauffée et agité jusqu'à obtention d'une émulsion claire rouge foncé.

9. Procédé selon l'une des revendications 5 à 8, **caractérisé en ce qu'**une agitation à une température de 90° Celsius est effectuée à l'étape b).

10. Procédé selon l'une des revendications 5 à 9, **caractérisé en ce qu'**une solution d'au moins un antioxydant est ajoutée à l'émulsion contenant du bêta-carotène obtenue à l'étape d) à une température d'environ 50°.

11. Procédé selon la revendication 10, **caractérisé en ce que** de l'acétylcystéine, en particulier de l'acétylcystéine dissoute dans de l'eau, est ajoutée à l'émulsion contenant l'antioxydant à une température de 30° Celsius.

12. Procédé selon l'une des revendications 5 à 11, **caractérisé en ce que** les étapes du procédé, à l'exception de la première étape a), sont exécutées sous atmosphère d'azote.
